# EUROPEAN PATENT SPECIFICATION

(11) **EP 3 711 733 B1**
(45) Date of publication and mention of the grant of the patent: **14.02.2024**
(21) Application number: 18893959.9
(22) Date of filing: 20.12.2018
(51) Int. Cl.: A61F 13/49, A61F 13/496

(54) **ABSORBENT ARTICLE**
ABSORBIERENDER ARTIKEL
ARTICLE ABSORBANT

(30) Priority: 28.12.2017 JP 2017253872
(43) Date of publication of application: 23.09.2020
(73) Proprietor: Unicharm Corporation, Shikokuchuo-shi, Ehime 799-0111 (JP)
(72) Inventor: DAIO, Mamoru, Kanonji-shi, Kagawa 769-1602 (JP); KUDO, Etsuko, Kanonji-shi, Kagawa 769-1602 (JP)
(74) Representative: Dolleymores
(86) International application number: PCT/JP2018/046963
(87) International publication number: WO 2019/131428

(56) References cited:
- EP-A1- 2 905 002
- WO-A1-2015/001915
- JP-A- 2015 085 089
- JP-A- 2015 202 246
- JP-A- 2016 036 519
- JP-A- 2016 036 519
- JP-A- 2016 116 657

## Description

### [TECHNICAL FIELD]

The present invention relates to an absorbent article such as a disposable diaper.

### [BACKGROUND ART]

The absorbent article described in Patent Literature 1 has an absorbent body and an exterior body arranged on a non-skin surface side of the absorbent body. The absorbent body has an absorbent core and a plurality of body sheets extending outside in a front-rear direction relative to the absorbent core. The exterior body has a plurality of exterior sheets, and an elastic member arranged between the exterior sheets and stretching in a width direction.

### [CITATION LIST]

### [PATENT LITERATURE]

### [Patent Literature 1] JP 2016-34341 A

WO 2015/001915 A1 discloses a pants-type article in which front and rear waist regions include non-expanding-and-contracting sheets and expanding and contracting sheets. The expanding and contracting sheets are positioned outside a liquid absorbent core in the vertical direction so as to overlap the front and rear edges of a liquid absorbent structure. The expanding and contracting sheets form a ring -shaped elasticated band on an inner surface of the chassis.

EP 2905002 A1 relates to an absorbent article such as a disposable diaper. The article has an absorbent main body, having an absorber, and an exterior body which overlaps with the absorber. An opening unit is provided in the overlap region. A finger can be inserted in the opening unit.

JP 2016 036519 A discloses an absorbent article comprising an absorbent main body and an exterior body. Non-bonded regions, in which the exterior body is not joined to the absorbent main body, are provided to the outer end sections of the absorbent main body in the front-rear direction.

### [SUMMARY OF INVENTION]

The elastic member of the absorbent article of Patent Literature 1 is arranged on a non-skin surface side of the absorbent body. Contraction of the elastic member causes the absorbent body to fit the human body. However, the region in which the absorbent body is arranged is larger in the number of sheets than the region in which only the exterior body is arranged, and the rigidity is easy to become high. Accordingly, there was a risk that the absorbent body does not sufficiently fit the human body by the elastic member.

Therefore, there is a desire for an absorbent article in which the absorbent body is easily fitted to the human body by an elastic member arranged on the non-skin surface side of the absorbent body.

The present invention provides the absorbent article of independent claim 1. The dependent claims specify preferred but optional features.

### [BRIEF DESCRIPTION OF DRAWINGS]

Fig. 1 is a front view of an absorbent article according to an embodiment.
Fig. 2 is a plan view of the absorbent article according to the embodiment.
Fig. 3 is a cross-sectional view along a cross section A-A of the absorbent article shown in Fig. 2.
Fig. 4 is a cross-sectional view along a cross section B-B of the absorbent article shown in Fig. 2.
Fig. 5 is a view schematically showing a cross section of the absorbent article according to the embodiment in a mounted state.

### [DESCRIPTION OF EMBODIMENTS]

### (1) Outline of Embodiment

According to the present specification and the accompanying drawings, at least the following matters are disclosed.

An absorbent article according to one aspect includes a front-rear direction, a width direction orthogonal to the front-rear direction, a front waistline region, a rear waistline region, a crotch region arranged between the front waistline region and the rear waistline region, an absorbent body having an absorbent core and arranged to straddle the crotch region, the front waistline region, and the rear waistline region, an exterior body arranged in at least the front waistline region and the rear waistline region on a non-skin surface side of the absorbent body, and an elastic member stretchable in the width direction arranged in the exterior body. An outer end part in the front-rear direction of the absorbent body is provided with a body non-bonding region in which the absorbent body and the exterior body are not bonded. The elastic member has a first elastic portion arranged to overlap the body non-bonding region in a thickness direction. At the outer end part, contraction force in the width direction of the body non-bonding region is higher than contraction force in the width direction of an adjacent region extending outside in the front-rear direction from an outer end edge of the body non-bonding region.

The body non-bonding region is lower in rigidity than the region where the absorbent body and the exterior body are bonded. Since the first elastic portion is arranged so as to overlap the body non-bonding region, the absorbent body easily contracts by the contraction of the first elastic portion. Furthermore, the contraction force in the width direction of the body non-bonding region is higher than the contraction force in the width direction of the adjacent region, and the body non-bonding region contracts more than the adjacent region does and is easily fitted to the human body. Since the body non-bonding region easily contracts, the outer end part of the absorbent body provided with the body non-bonding region can be fitted to the human body.

According to a preferred aspect, at least a part of the body non-bonding region is arranged to overlap the absorbent core in the front-rear direction.

The region arranged to overlap the absorbent core in the front-rear direction easily becomes less likely to contract due to the rigidity of the absorbent core. By providing the body non-bonding region in at least a part of the region, the outer end part of the absorbent body can be fitted to the human body while suppressing the influence of the absorbent core.

According to a preferred aspect, the absorbent body has a central region which is a region obtained by dividing a total length of the absorbent core in the width direction into three equal parts and is located at a center of the absorbent core in the width direction. The body non-bonding region is arranged to overlap the central region in the front-rear direction.

The force easily concentrates on the central region of the absorbent core in the width direction when sandwiched between the legs or the like, and the region arranged to overlap the central region in the front-rear direction easily becomes less likely to contract due to the rigidity of the absorbent core. By providing the body non-bonding region in at least a part of the region, the outer end part of the absorbent body can be fitted to the human body while suppressing the influence of the absorbent core.

According to a preferred aspect, the body non-bonding region is arranged to overlap, in the front-rear direction, an entire region of the absorbent core in the width direction.

By providing the body non-bonding region in the region arranged to overlap, in the front-rear direction, the absorbent core in the width direction, the outer end part of the absorbent body can be fitted to the human body while suppressing the influence of the absorbent core.

According to a preferred aspect, an end edge in the crotch region side of the body non-bonding region is arranged so as not to overlap the absorbent core in planar view of a thickness direction.

The body non-bonding region is less likely to be affected by the absorbent core and becomes easy to contract in the width direction. Stretching of the first elastic portion causes the outer end part of the absorbent body to become more easily fitted to the human body.

According to a preferred aspect, the elastic member has a second elastic portion arranged on the side of the crotch region relative to the body non-bonding region and outside in the front-rear direction relative to the absorbent core. The second elastic portion is curved to the side of the crotch region.

The second elastic portion allows the outer end part of the absorbent body to be pulled up to a waistline opening side. By pulling up the absorbent body to the waistline opening side, the absorbent article becomes less likely to be pulled down, and the fitness becomes easily maintained. In the configuration in which the body non-bonding region and the second elastic portion are provided in the front waistline region, the absorbent article can be pulled up with respect to a swelling of the abdomen of the wearer, and it becomes easy to continuously cover the abdomen.

According to a preferred aspect, a plurality of the first elastic portions is provided at intervals in the front-rear direction.

The plurality of first elastic portions allows the entire region where the first elastic portions are arranged to be fitted to the human body. Compared with a configuration in which the absorbent body is fitted to the human body by a single first elastic portion, it becomes less likely to locally adhere, thereby allowing the contact with the skin to be softened.

According to a preferred aspect, a first elastic portion having a highest contraction force of the first elastic portions is arranged so as not to overlap the absorbent core in the thickness direction.

The first elastic portion having the highest contraction force does not overlap the absorbent core, and the contractility is difficult to be inhibited by the rigidity of the absorbent core. Hence, the outer end part of the absorbent body is easily fitted to the human body by the contraction of the first elastic portion having the highest contraction force.

According to a preferred aspect, the absorbent body has a plurality of sheet materials extending outside in the front-rear direction relative to the absorbent core. The outer end part of the absorbent body is provided with a sheet non-bonding region in which at least two sheet materials are not bonded together. The sheet non-bonding region, the body non-bonding region. The first elastic portion are arranged to overlap in the thickness direction at the outer end part.

Since the sheet non-bonding region, the body non-bonding region, and the first elastic portion are arranged to overlap, the rigidity of the region where the first elastic portion is arranged becomes lower, and the first elastic portion becomes more easily to contract. Hence, the outer end part of the absorbent body is more easily to be fitted to the human body.

According to a preferred aspect, the exterior body has a plurality of exterior sheets. An outside in the front-rear direction relative to an outer end edge of the absorbent body is provided with an exterior non-bonding region in which at least two exterior sheets of the exterior sheets are not bonded together, and
the exterior non-bonding region, the body non-bonding region. The first elastic portion are arranged to overlap in the thickness direction at the outer end part.

Since the exterior non-bonding region, the body non-bonding region, and the first elastic portion are arranged to overlap, the rigidity of the region where the first elastic portion is arranged becomes lower, and the first elastic portion becomes more easily to contract. Hence, the outer end part of the absorbent body is more easily to be fitted to the human body.

A body bonding region in which the absorbent body and the exterior body are bonded is arranged outside in the width direction relative to the body non-bonding region at the outer end part. The elastic member has a third elastic portion arranged to overlap the body bonding region in the thickness direction. The third elastic portion is curved to the side of the crotch region.

Since the third elastic portion overlapping the body bonding region is curved to the crotch region side, it is possible to pull up the exterior body by the third elastic portion and to transmit the pulling force by the third elastic portion also to the absorbent body. By pulling up the absorbent body to the waist opening side, the absorbent article becomes less likely to be pulled down, and the fitness becomes easily maintained. In the configuration in which the body non-bonding region and the third elastic portion are provided in the front waistline region, the absorbent article can be pulled up with respect to a swelling of the abdomen of the wearer, and it becomes easy to continuously cover the abdomen.

### (2) Absorbent article according to the first embodiment

The absorbent article according to the embodiment will be described below with reference to the drawings. It is to be noted that in the following drawings, identical or similar parts are denoted by the identical or similar reference numerals. However, it is to be noted that the drawings are schematic, and the proportions of each dimension are different from the actual ones. Accordingly, specific dimensions should be determined in consideration of the following explanation. It is also possible to include parts having different dimensional relationships and proportions among the drawings.

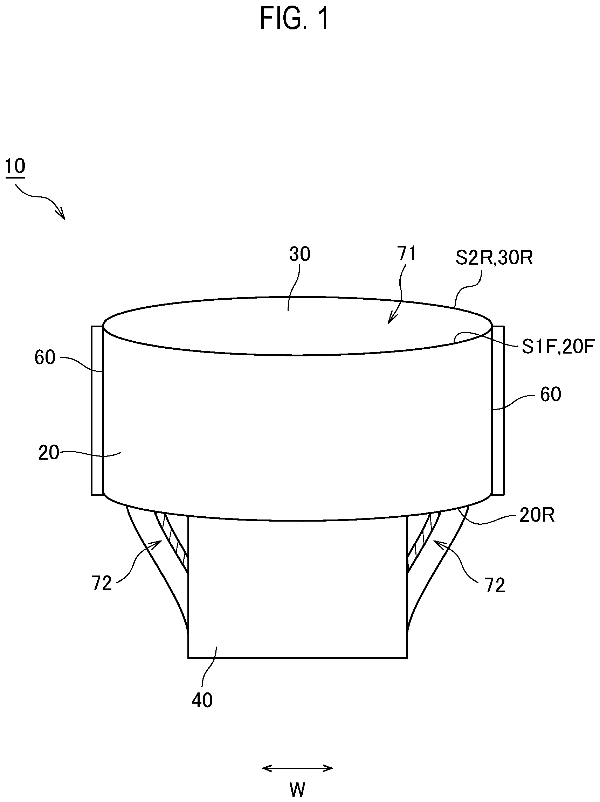

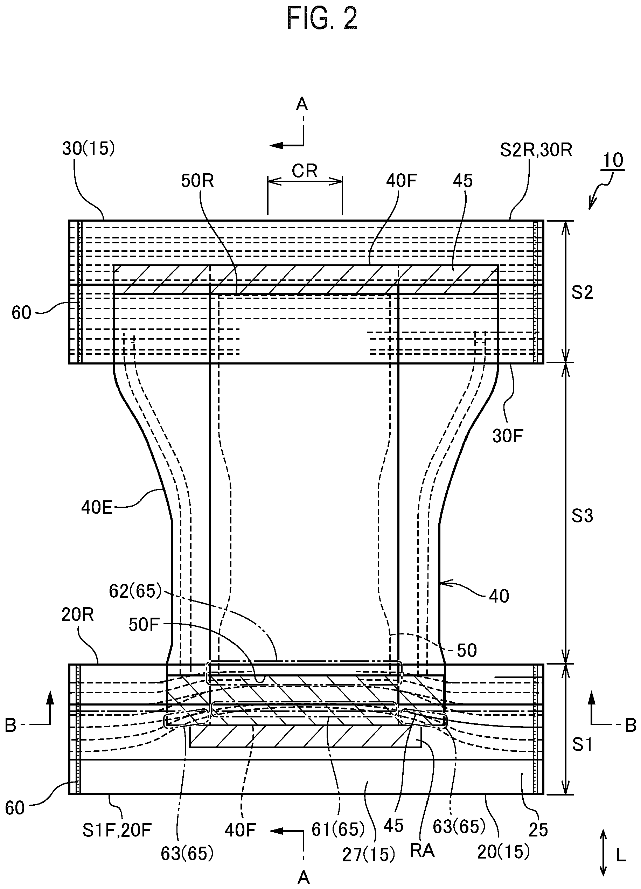

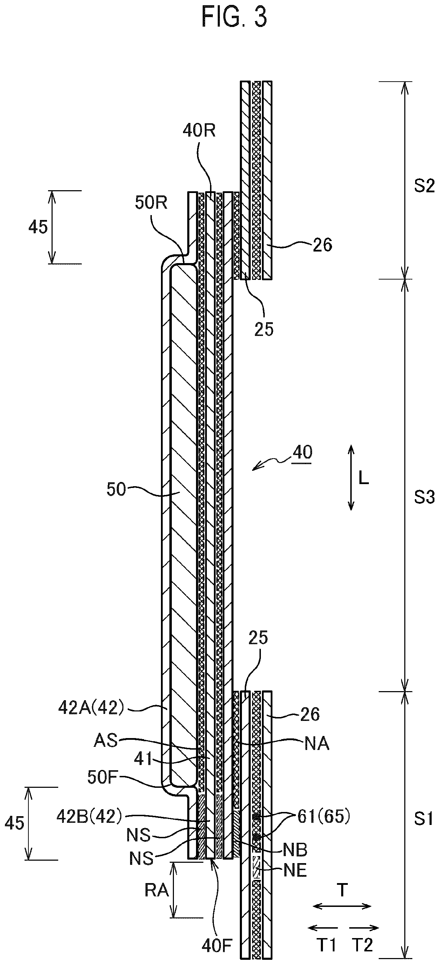

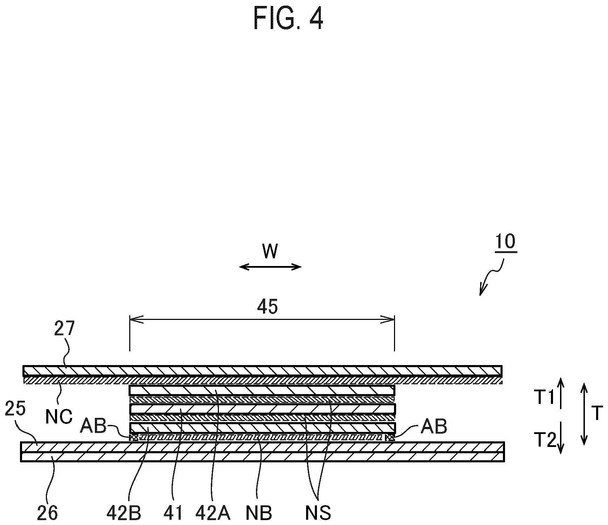

The absorbent article may also be a tape type or an underpants type. The absorbent article may be a disposable diaper or a shorts type sanitary napkin. The absorbent article of the first embodiment is an underpants type disposable diaper. Fig. 1 is a schematic front view of an absorbent article 10 according to the present embodiment. Fig. 2 is a schematic plan view of the absorbent article 10 according to the present embodiment. The schematic plan view shown in Fig. 2 shows a stretched state in which the absorbent article 10 is stretched to a state in which no wrinkles are formed in a state where a side bonded portion 60 described later is developed. Fig. 3 is a cross-sectional view along a line A-A shown in Fig. 2, and Fig. 4 is a cross-sectional view along a line B-B shown in Fig. 2.

The absorbent article 10 has a front-rear direction L and a width direction W that are orthogonal to each other. The front-rear direction L is defined by the direction extending to a front side of the human body and a rear side of the human body. In other words, the front-rear direction L is a direction extending back and forth of the developed absorbent article 10. In addition, the absorbent article 10 has a thickness direction T orthogonal to both the front-rear direction L and the width direction W

The absorbent article 10 has a front waistline region S1, a rear waistline region S2, and a crotch region S3. The front waistline region S1 is a region facing the front waistline (abdomen) of the wearer. The rear waistline region S2 is a region facing the rear waistline (back) of the wearer. The crotch region S3 is a region located at the crotch of the wearer and arranged between the front waistline region S1 and the rear waistline region S2.

In the present embodiment, the absorbent article 10 may have an exterior body 15 and an absorbent body 40. The exterior body 15 overlaps the absorbent body 40 in the thickness direction and is arranged in at least the front waistline region S1. The exterior body 15 may have a front exterior body 20 and a rear exterior body 30. The front exterior body 20 is an exterior body arranged on a non-skin surface side T2 of the absorbent body 40 in the front waistline region S1. The rear exterior body 30 is an exterior body separated from the front exterior body 20 in the front-rear direction L and arranged on the non-skin surface side T2 of the absorbent body 40 in the rear waistline region S2.

The crotch region S3 is a region in which a leg opening 72 (See Fig. 1) described later is formed. A boundary on the crotch region side of the front waistline region S1 is a front end edge of the leg opening 72, and a boundary on the crotch region side of the rear waistline region S2 is a rear end edge of the leg opening 72. In another embodiment, the exterior body 15 may be configured by integrating the front exterior body 20 and the rear exterior body 30 and provided from the front waistline region S1 to the rear waistline region S2.

The front exterior body 20 and the rear exterior body 30 may be constituted of a sheet such as a nonwoven fabric. The front exterior body 20 may have a plurality of exterior sheets. The exterior sheet may have a first exterior sheet 25 and a second exterior sheet 26 located on the non-skin surface side T2 of the first exterior sheet 25.

The exterior body 15 may have an elastic member 65 stretchable in the width direction. The elastic member 65 may be configured by, for example, a rubber thread stretchable in the width direction W, or an elastic sheet stretchable in the width direction W The elastic member 65 of the present embodiment is configured by a rubber thread, and is arranged between the first exterior sheet 25 and the second exterior sheet 26 in the thickness direction T. A plurality of the elastic members 65 is arranged at intervals in the front-rear direction L. The elastic members 65 may be linearly arranged along the width direction W, or may be arranged to be inclined in the width direction W

The elastic member 65 includes at least a first elastic portion 61, a second elastic portion 62, and a third elastic portion 63, which will be described later. The first elastic portion 61, the second elastic portion 62, and the third elastic portion 63 are parts of the elastic member and are portions stretchable in the width direction. The first elastic portion 61, the second elastic portion 62, and the third elastic portion 63 may be parts of the same elastic member or may be parts of elastic members 65 different from one another. The first elastic portion 61, the second elastic portion 62, and the third elastic portion 63 may be provided in each of the plurality of elastic members, or may be provided in a single elastic member.

As shown in Fig. 1, a side bonded portion 60 in which an outside part of the front waistline region S1 in the width direction W and an outside part of the rear waistline region S2 in the width direction W are bonded may be provided. Fig. 2 shows a state in which the bonding at the side bonded portion 60 is released and the absorbent article 10 is developed. The side bonded portion 60 may extend along the front-rear direction L in each of the front exterior body 20 and the rear exterior body 30.

It is to be noted that in the present invention, the outside part is a part occupying a certain range in a width direction W including an outer edge in the width direction W, and an outside edge is the outer edge in the width direction W In the present invention, the inside part is a part occupying a certain range in the width direction W including an inner edge in the width direction W, and an inside edge is the inner edge in the width direction W In addition, the front end part and the rear end part in the present invention are parts occupying a certain range in the front-rear direction L including the edge in the front-rear direction L, and the front end edge and the rear end edge are edges in the front-rear direction L. An outer end part includes the front end part and the rear end part, and an outer end edge includes the front end edge and the rear end edge.

As shown in Fig. 1, in a state where the side bonded portion 60 is formed, the absorbent article 10 is formed with a waistline opening 71 through which the waist of the wearer passes, and a pair of the leg openings 72 into which the legs of the wearer are inserted. The waistline opening 71 may be defined by a front end edge 20F of the front exterior body that is a front end edge S1F of the front waistline region S1 and a rear end edge 30R of the rear exterior body 30 that is a rear end edge S2R of the rear waistline region S2. In addition, the leg opening 72 may be defined by a rear end edge 20R of the front exterior body 20 extending outward in the width direction W relative to the absorbent body 40, a front end edge 30F of the rear exterior body 30 extending outward in the width direction W relative to the absorbent body 40, and an outside edge 40E of the absorbent body 40 in the crotch region S3.

The absorbent body 40 is arranged to straddle the front exterior body 20 and the rear exterior body 30. That is, the absorbent body 40 extends over the front waistline region S1, the rear waistline region S2, and the crotch region S3. The absorbent body 40 may be configured as a separate body from the front exterior body 20 and the rear exterior body 30. The absorbent body 40 may have a region in which the front exterior body 20 or the rear exterior body 30 is arranged on the non-skin surface side T2, and a region which does not overlap the exterior body 15 in the thickness direction T.

The absorbent body 40 includes at least an absorbent core 50. The absorbent core 50 may include, for example, ground pulp or a superabsorbent polymer (SAP), or a mixture of them. The absorbent core 50 is arranged in at least the crotch region S3. Preferably, the absorbent core 50 may extend from the front waistline region S1 to the rear waistline region S2 in the front-rear direction L. The absorbent core 50 may be covered with a core wrap.

As shown in Fig. 2, the absorbent body 40 may have an outer end part 45 extending to the front side relative to the absorbent core 50. The outer end part 45 is a region between a front end edge 50F of the absorbent core 50 and a front end edge 40F of the absorbent body 40 in the front-rear direction L, and a region between a rear end edge 50R of the absorbent core 50 and a rear end edge 40R of the absorbent body 40 in the front-rear direction L. The outer end part 45 is a region from one outside edge 40E of the absorbent body 40 to the other outside edge 40E in the width direction W Figs. 3 and 4 show the range of the outer end part 45 in the front-rear direction and the range of the outer end part 45 in the width direction. In addition, the absorbent body 40 may have a central region CR located at the center in the width direction W of the absorbent core 50. The central region CR is a region located at the center in the width direction W of the region obtained by dividing the total length of the absorbent core 50 in the width direction W into three equal parts.

As shown in Figs. 3 and 4, the absorbent body 40 may include at least a liquid-impermeable sheet 41 and a body sheet 42 overlapping the liquid-impermeable sheet 41 in the thickness direction T. The liquid-impermeable sheet 41 and the body sheet 42 may be arranged in at least the outer end part 45. The liquid-impermeable sheet 41 and the body sheet 42 may be arranged to straddle a region overlapping the absorbent core 50 and the outer end part 45. The liquid-impermeable sheet 41 may be arranged on the non-skin surface side T2 relative to the absorbent core 50. The body sheet 42 may have a first body sheet 42A arranged on the skin surface side T1 relative to the liquid-impermeable sheet 41 and on the skin surface side T1 relative to the absorbent core 50, and a second body sheet 42B arranged on the non-skin surface side T2 relative to the liquid-impermeable sheet 41.

In the outer end part 45 of the absorbent body 40 of the present embodiment, the first body sheet 42A, the liquid-impermeable sheet 41, and the second body sheet 42B are arranged in this order from the skin surface side T1 toward the non-skin surface side T2. In the region overlapping the absorbent core 50 of the present embodiment, the first body sheet 42A, the absorbent core 50, the liquid-impermeable sheet 41, and the second body sheet 42B are arranged in this order from the skin surface side T1 toward the non-skin surface side T2. The first body sheet 42A and the liquid-impermeable sheet 41 may be arranged to sandwich the absorbent core 50 in the thickness direction T.

The liquid-impermeable sheet 41 is only required to be liquid-impermeable, and may be constituted of, for example, a film. The liquid-impermeable sheet 41 may be constituted of a single sheet. Alternatively, the liquid-impermeable sheet 41 may be constituted of a laminated sheet in which a plurality of sheets are laminated to one another. In this case, at least one of the plurality of sheets may have liquid-impermeability.

The body sheet 42 is only required to be liquid-permeable, and may be constituted of, for example, a nonwoven fabric. The body sheet 42 may be constituted of a single sheet. Alternatively, the body sheet 42 may be constituted of a laminated sheet in which a plurality of sheets are laminated to one another.

The absorbent article thus configured has a bonding region and a non-bonding region. The bonding region includes a region where the exterior body 15 and the absorbent body 40 are bonded, a region where the liquid-impermeable sheet 41 and the body sheet 42 of the absorbent body 40 are bonded, and a region where the exterior sheets of the exterior body are bonded together. The non-bonding region includes a region where the exterior body 15 and the absorbent body 40 are not bonded, a region where the liquid-impermeable sheet 41 and the body sheet 42 of the absorbent body 40 are not bonded, and a region where the exterior sheets of the exterior body are not bonded together. Next, the bonding region and the non-bonding region of the absorbent article 10 will be described. It is to be noted that the non-bonding region in the present invention includes not only a region where no adhesive is applied but also a region where an adhesive is slightly applied but the members are not bonded via the adhesive.

The absorbent article 10 has a body non-bonding region NB where the absorbent body 40 and the exterior body 15 are not bonded. The body non-bonding region NB may be a region in which, among the sheets constituting the absorbent body 40, the sheet arranged to face the exterior body 15 and the exterior body 15 are not bonded. Specifically, in the form where the second body sheet 42B is arranged on the entire surface of the non-skin surface of the absorbent body 40, the body non-bonding region NB may be a region where the second body sheet 42B and the exterior body 15 are not bonded. In the form where the second body sheet 42B and the liquid-impermeable sheet 41 are arranged on the non-skin surface of the absorbent body 40, the body non-bonding region NB may include a region in which the second body sheet 42B and the exterior body 15 are not bonded, and a region in which the liquid-impermeable sheet 41 and the exterior body 15 are not bonded.

The first elastic portion 61 of the elastic member 65 overlaps the body non-bonding region NB. The first elastic portion 61 may overlap at least a part of the body non-bonding region NB. The body non-bonding region NB stretches in the width direction W by the first elastic portion 61 and is fitted to the human body. The contraction force in the width direction W of the body non-bonding region NB may be higher than the contraction force in the width direction W of an adjacent region RA extending outside in the front-rear direction L from the outer end edge of the body non-bonding region NB. The adjacent region RA is a region extending outside in the front-rear direction L from the outer end edge of the body non-bonding region NB.

The contraction force can be measured by the following method. First, a test piece to be measured is prepared. In the case where the absorbent article has a side bonded portion, the side bonded portion is released to develop the absorbent article. Next, in a maximum stretched state where the absorbent article is extended so as to be free from wrinkles or minimize wrinkles, the length in the front-rear direction of the region in which the body non-bonding region NB is provided is measured, and a mark is given to a position shifted, by its length, from the outer end edge of the body non-bonding region to the outside of the front-rear direction L. The region between this mark and the outer end edge of the body non-bonding region is an adjacent region. The length of the adjacent region in the width direction is the same as the length of the body non-bonding region in the width direction. The region where the body non-bonding region is provided and the adjacent region are cut from the absorbent article. Next, in a state where the test piece of each region having been cut is sufficiently contracted, both end parts of the test piece are held by a chuck (holding tool) of the tensile tester. Next, in a state where one of the chucks in the width direction W is fixed, the other chuck is reciprocated by one reciprocation so as to change the distance between the chucks. The moving speed of the chuck at this time is 300 mm/min. It is to be noted that the timing to change the moving direction of the chuck is a point of 95% at the time of maximum stretch. The stress applied on the chuck is measured while the chuck is in motion. The stress obtained at 65% from the time of maximum stretch in a return of the one reciprocation is defined as "contraction force".

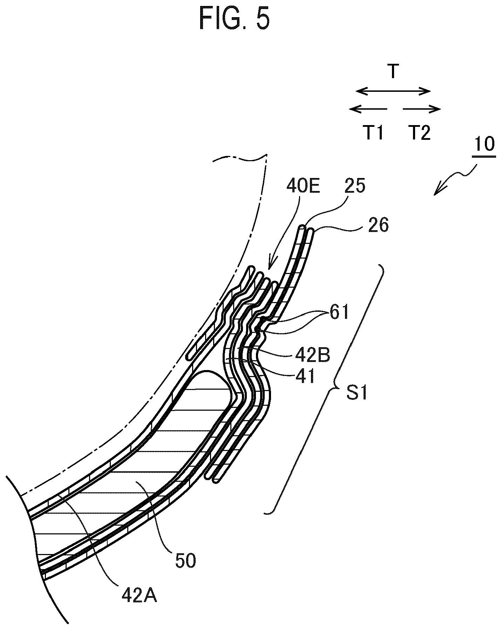

According to the absorbent article 10 thus configured, the absorbent body 40 becomes easily fitted to the human body by the elastic member 65 arranged on the non-skin surface side T2 of the absorbent body 40. Fig. 5 is a view schematically showing a cross section of the absorbent article in a mounted state, and schematically shows the abdomen of the wearer and the front waistline region of the absorbent article. The dashed lines shown in Fig. 5 indicate the body line of the wearer. The outer end part 45 of the absorbent article 10 is provided with the body non-bonding region NB. The body non-bonding region NB is lower in rigidity than the region where the absorbent body 40 and the exterior body 15 are bonded. Since the first elastic portion 61 is arranged so as to overlap the body non-bonding region NB, the absorbent body 40 easily contracts by the contraction of the first elastic portion 61. The contraction force in the width direction W of the body non-bonding region NB is higher than the contraction force in the width direction of the adjacent region RA, and the body non-bonding region NB contracts more than the adjacent region RA does and is easily fitted to the human body. Since the body non-bonding region NB easily contracts, the outer end part 45 of the absorbent body 40 provided with the body non-bonding region NB can be fitted to the human body.

The body non-bonding region NB may be provided in the entire region of the outer end part 45 or may be provided in a part of the outer end part 45. The outer end edge of the body non-bonding region NB coincides with the outer end edge of the absorbent body 40. The inner end edge of the body non-bonding region NB in the front-rear direction may be located outside in the front-rear direction relative to the outer end edge of the absorbent core 50, or may coincide with the outer end edge of the absorbent core 50. The outside edge of the body non-bonding region NB may coincide with the outside edge 40E of the absorbent body 40, or may be located inside in the width direction W relative to the outside edge 40E of the absorbent body 40. It is to be noted that while the body non-bonding region NB and the elastic member 65 of the present embodiment are provided only in the front waistline region S1, they may similarly be provided in the rear waistline region S2.

Preferably, at least a part of the body non-bonding region NB may be arranged to overlap the absorbent core 50 in the front-rear direction L. The region arranged to overlap the absorbent core 50 in the front-rear direction L is easy to be subjected to a force directed from the absorbent core 50 to the front side. By providing the body non-bonding region NB in at least a part of the region, the outer end part 45 of the absorbent body 40 can be fitted to the human body while suppressing the influence of the absorbent core.

More preferably, the body non-bonding region NB may be arranged to overlap the central region CR in the front-rear direction L. The force easily concentrates on the central region CR of the absorbent core 50 in the width direction when sandwiched between the legs or the like, and the region arranged to overlap the central region CR in the front-rear direction L may become less likely to contract due to the rigidity of the absorbent core 50. By providing the body non-bonding region NB in at least a part of the region, the outer end part 45 of the absorbent body 40 can be fitted to the human body while suppressing the influence of the absorbent core 50.

More preferably, the body non-bonding region NB may be arranged to overlap, in the front-rear direction, the entire region in the width direction W of the absorbent core 50. By providing the body non-bonding region NB in the region arranged to overlap, in the front-rear direction L, the absorbent core 50 in the width direction W, the outer end part 45 of the absorbent body 40 can be fitted to the human body while suppressing the influence of the absorbent core.

The end edge in the crotch region side of the body non-bonding region NB may be arranged so as not to overlap the absorbent core 50 in the thickness direction. That is, the body non-bonding region NB and the absorbent core 50 may be separated in the front-rear direction L or the width direction W The body non-bonding region NB is less likely to be affected by the absorbent core 50 and becomes more likely to contract in the width direction W, and the outer end part becomes more easily fitted to the human body.

A plurality of the first elastic portions 61 of the elastic member 65 may be provided at intervals in the front-rear direction L. The plurality of first elastic portions 61 allows the entire region where the first elastic portions 61 are arranged to be fitted to the human body. Compared with a configuration in which the absorbent body is fitted to the human body by a single first elastic portion 61, it becomes less likely to locally adhere, thereby allowing the contact with the skin to be softened.

The first elastic portion 61 having the highest contraction force of the first elastic portions 61 may be arranged so as not to overlap the absorbent core 50 in the thickness direction T. The first elastic portion 61 having the highest contraction force does not overlap the absorbent core 50, and the contractility is difficult to be inhibited by the rigidity of the absorbent core. Hence, the outer end part of the absorbent body 40 is easily fitted to the human body by the contraction of the first elastic portion 61 having the highest contraction force.

The elastic member 65 may have the second elastic portion. The second elastic portion 62 is arranged on the crotch region side relative to the first elastic portion. The second elastic portion 62 may be arranged on the crotch region side relative to the body non-bonding region NB and outside in the front-rear direction L relative to the absorbent core 50. The second elastic portion 62 may be curved to the crotch region side. That is, the second elastic portion 62 may extend from the outside in the width direction W toward the inside in the width direction W toward the inside in the front-rear direction. The second elastic portion 62 is arranged in a region between the body non-bonding region and the absorbent core in the front-rear direction L, and contracts the region in the width direction and contracts it toward the front-rear direction. The second elastic portion allows the outer end part of the absorbent body to be pulled up to the waistline opening side. By pulling up the absorbent body to the waistline opening side, the absorbent article becomes less likely to be pulled down, and the fitness becomes easily maintained. Furthermore, in the configuration in which the body non-bonding region and the second elastic portion are provided in the front waistline region, the absorbent article can be pulled up with respect to a swelling of the abdomen of the wearer, and it becomes easy to continuously cover the abdomen.

The absorbent article 10 is provided with a body bonding region AB in which the absorbent body 40 and the exterior body 15 are bonded. As shown in Fig. 4, the body bonding region AB is arranged outside in the width direction W relative to the body non-bonding region NB at the outer end part 45. The body bonding region AB may be arranged on both sides in the width direction of the body non-bonding region NB, and may be provided in a region extending inside from the outside edge of the absorbent body 40. The third elastic portion 63 of the elastic member 65 is arranged to overlap the body bonding region AB in the thickness direction T. The third elastic portion 63 is curved to the crotch region side. That is, the third elastic portion 63 may extend from the outside in the width direction toward the inside in the width direction toward the inside in the front-rear direction. The third elastic portion 63 is located on the most crotch region side in the center of the width direction of the absorbent body 40. Since the third elastic portion 63 overlaps the body bonding region AB and the third elastic portion 63 is curved to the crotch region side, it is possible to pull up the exterior body 15 by the third elastic portion 63 and to transmit the pulling force by the third elastic portion 63 also to the absorbent body 40. By pulling up the absorbent body 40 to the waistline opening 71 side, the absorbent article 10 becomes less likely to be pulled down, and the fitness becomes easily maintained. In the configuration in which the body non-bonding region NB and the third elastic portion 63 are provided in the front waistline region S1, the absorbent article can be pulled up with respect to a swelling of the abdomen of the wearer, and it becomes easy to continuously cover the abdomen.

The third elastic portion 63 may be arranged outside in the width direction relative to the first elastic portion 61. The third elastic portion 63 may be a part of the same elastic member as the first elastic portion or may be a part of the same elastic member as the second elastic portion.

The absorbent article 10 may have a sheet non-bonding region NS where at least two sheet materials of the plurality of body sheets are not bonded together. At least a part of the sheet non-bonding region NS may be provided at the outer end part 45. The sheet non-bonding region NS may be provided in the entire region of the outer end part 45 or may be provided in a part of the outer end part 45. The sheet non-bonding region NS may include a region in which the liquid-impermeable sheet 41 and the body sheet 42 are not bonded and a region in which the body sheets 42 are not bonded together.

The sheet non-bonding region NS, the body non-bonding region NB, and the first elastic portion 61 may be arranged to overlap in the thickness direction T at the outer end part 45. The outer end part 45 is provided with a region where the sheet materials constituting the absorbent body 40 are not bonded together and the absorbent body 40 and the exterior body 15 are not bonded. Since the outer end part 45 of the absorbent body 40 is provided with the sheet non-bonding region NS, the rigidity of the outer end part 45 of the absorbent body 40 becomes lower than that of the configuration in which all the body sheets are bonded, and the outer end part 45 of the absorbent body 40 is easy to deform. In particular, the liquid-impermeable sheet 41 is constituted of a film or the like, and is often higher in rigidity than a liquid-permeable sheet. The sheet non-bonding region NS can reduce the rigidity due to the liquid-impermeable sheet 41, and the outer end part 45 of the absorbent body 40 becomes easy to deform. Since the sheet non-bonding region NS, the body non-bonding region, and the first elastic portion overlap at the outer end part 45, the outer end part 45 becomes more likely to contract by the first elastic portion, and the outer end part becomes more easily fitted to the human body.

The outer end edge of the sheet non-bonding region NS may coincide with the outer end edge of the absorbent body 40, or may be located inside in the front-rear direction relative to the outer end edge of the absorbent body 40. The inner end edge of the sheet non-bonding region NS may be located outside in the front-rear direction relative to the outer end edge of the absorbent core 50, or may coincide with the outer end edge of the absorbent core 50. The outside edge of the sheet non-bonding region NS may coincide with the outside edge 40E of the absorbent body 40, or may be located inside in the width direction W relative to the outside edge 40E of the absorbent body 40.

The absorbent article 10 may have a sheet bonding region AS in which the liquid-impermeable sheet 41 and the body sheet 42 are bonded on the crotch region side relative to the sheet non-bonding region NS. The sheet bonding region AS is adjacent to the sheet non-bonding region NS in the front-rear direction. The sheet bonding region AS is a region where sheets not bonded in the sheet non-bonding region NS are bonded together.

In a form where the sheet non-bonding region NS is provided between the sheet materials sandwiching the absorbent core 50 in the thickness direction and straddling the absorbent core 50 and the outer end part 45, the inner end edge of the sheet non-bonding region NS on the crotch region side, i.e., the inner end edge of the sheet bonding region AS may be arranged outside in the front-rear direction relative to the absorbent core 50. Since the inner end edge of the sheet non-bonding region NS is located outside in the front-rear direction relative to the absorbent core 50, the sheet bonding region AS is provided outside in the front-rear direction relative to the absorbent core 50. It is possible to suppress leakage of an absorptive material constituting the absorbent core 50 to the outside of the absorbent body 40 by the sheet bonding region AS.

The sheet non-bonding region NS may be any of a region where the liquid-impermeable sheet 41 is not bonded to the first body sheet 42A, a region where the liquid-impermeable sheet 41 is not bonded to the second body sheet 42B, and a region where the liquid-impermeable sheet 41 is not bonded to the first body sheet 42A and the second body sheet 42B.

The positional relationship in the front-rear direction between the sheet non-bonding region NS and the body non-bonding region NB is not limited. The front end edge of the sheet non-bonding region NS may be on the front side relative to the front end edge of the body non-bonding region NB, or may be on the rear side relative to the front end edge of the body non-bonding region NB. The rear end edge of the sheet non-bonding region NS may be on the front side relative to the rear end edge of the body non-bonding region NB, or may be on the rear side relative to the rear end edge of the body non-bonding region NB. Preferably, the sheet non-bonding region NS may extend to the crotch region side relative to the body non-bonding region NB. The liquid-impermeable sheet 41 is often higher in rigidity than the liquid-permeable sheet, and the sheet non-bonding region NS is more likely to have an effect of lowering the rigidity than the body non-bonding region NB has. Since the sheet non-bonding region NS extends to the crotch region side relative to the body non-bonding region NB, the outer end part of the absorbent body 40 becomes easier to deform, and the outer end part becomes more easily fitted to the human body.

The positional relationship in the width direction W between the sheet non-bonding region NS and the body non-bonding region NB is not limited. The outer end edge of the sheet non-bonding region NS may be outside relative to the outside edge of the body non-bonding region NB, or may be inside relative to the outside edge of the body non-bonding region NB. Preferably, the sheet non-bonding region NS may extend outside in the width direction W relative to the body non-bonding region NB.

The absorbent article 10 may have an exterior non-bonding region NE. The exterior non-bonding region NE is a region where the exterior sheets are not bonded together. The exterior non-bonding region NE may be provided outside in the front-rear direction relative to the outer end edge of the absorbent body 40, or may be provided so as to overlap the absorbent body 40. The exterior non-bonding region NE, the body non-bonding region NB, and the first elastic portion 61 may be arranged to overlap in the thickness direction T at the outer end part. Since the exterior non-bonding region NE, the body non-bonding region NB, and the first elastic portion 61 are arranged to overlap at the outer end part 45, the outer end part 45 becomes more likely to contract by the first elastic portion, and the outer end part becomes more easily fitted to the human body.

While the present invention has been described in detail with reference to the above-described embodiment, it is obvious to those skilled in the art that the present invention is not limited to the embodiment described in the present description. The present invention can be implemented as modifications and variations without departing from the scope of the present invention set forth by the claims. Accordingly, the description of the present description is intended to be illustrative and has no restrictive meaning to the present invention.

### [INDUSTRIAL APPLICABILITY]

It is possible to provide an absorbent article in which the absorbent body is easily fitted to the human body by an elastic member arranged on the non-skin surface side of the absorbent body.

### [REFERENCE SIGNS LIST]

- 10: Absorbent article
- 15: Exterior body
- 20: Front exterior body
- 30: Rear exterior body
- 40: Absorbent body
- 41: Liquid-impermeable sheet
- 42A: First body sheet (body sheet)
- 42B: Second body sheet (body sheet)
- 45: Outer end part
- 50: Absorbent core
- 60: Side bonded portion
- 65: elastic member
- 61: first elastic portion
- AB: Body bonding region
- AS: Sheet bonding region
- NB: Body non-bonding region
- NE: Exterior non-bonding region
- NS: Sheet non-bonding region
- S1: Front waistline region
- S2: Rear waistline region
- S3: Crotch region
- L: Front-rear direction
- W: Width direction

## Claims

1. An absorbent article (10), comprising:
a front-rear direction (L);
a width direction (W) orthogonal to the front-rear direction;
a front waistline region (S1);
a rear waistline region (S2);
a crotch region (S3) arranged between the front waistline region and the rear waistline region;
an absorbent body (40) having an absorbent core (50) and arranged to straddle the crotch region, the front waistline region, and the rear waistline region;
an exterior body (15) arranged in at least the front waistline region (S1) and the rear waistline region (S2) on a non-skin surface side of the absorbent body (40); and
an elastic member (65) stretchable in the width direction arranged in the exterior body (15), wherein
an outer end part (45) includes a front end part and a rear end part,
the front end part is a region between a front end edge (50F) of the absorbent core (50) and a front end edge (40F) of the absorbent body (40) in the front-rear direction (L) and is a region from one outside edge (40E) of the absorbent body (40) to the other outside edge (40E) in the width direction (W),
the rear end part is a region between a rear end edge (50R) of the absorbent core (50) and a rear end edge (40R) of the absorbent body (40) in the front-rear direction (L), and is a region from one outside edge (40E) of the absorbent body (40) to the other outside edge (40E) in the width direction (W),
one or both of the front end part and the rear end part of the outer end part (45) in the front-rear direction of the absorbent body (40) is provided with a body non-bonding region (NB) in which the absorbent body (40) and the exterior body (15) are not bonded, the outer end edge of the body non-bonding region (NB) coincides with the outer end edge of the absorbent body (40) in the front-rear direction (L),
the elastic member (65) has a first elastic portion (61) arranged to overlap the body non-bonding region (NB) in a thickness direction,
at the outer end part (45), contraction force in the width direction of the body non-bonding region (NB) is higher than contraction force in the width direction of an adjacent region extending outside in the front-rear direction from an outer end edge of the body non-bonding region (NB),
the absorbent article (10) is provided with a body bonding region (AB) in which the absorbent body (40) and the exterior body (15) are bonded,
the body bonding region (AB) is arranged outside in the width direction (W) relative to the body non-bonding region (NB) at the outer end part (45), and
the elastic member (65) has a third elastic portion (63) arranged to overlap the body bonding region (AB) in the thickness direction, and
the third elastic portion (63) is curved to the side of the crotch region (S3).

2. The absorbent article according to claim 1, wherein at least a part of the body non-bonding region (NB) is arranged to overlap the absorbent core (50) in the front-rear direction.

3. The absorbent article according to claim 2, wherein
the absorbent body (40) has a central region (CR) which is a region obtained by dividing a total length of the absorbent core (50) in the width direction into three equal parts and is located at a center of the absorbent core in the width direction, and
the body non-bonding region (NB) is arranged to overlap the central region (CR) in the front-rear direction.

4. The absorbent article according to claim 2 or 3, wherein the body non-bonding region (NB) is arranged to overlap, in the front-rear direction, an entire region of the absorbent core (50) in the width direction.

5. The absorbent article according to claim 1, wherein an end edge in the crotch region side of the body non-bonding region (NB) is arranged so as not to overlap the absorbent core (50) in planar view of a thickness direction (T).

6. The absorbent article according to claim 5, wherein
the elastic member (65) has a second elastic portion (62) arranged on the side of the crotch region (S3) relative to the body non-bonding region (NB) and outside in the front-rear direction relative to the absorbent core (50), and
the second elastic portion (62) is curved to the side of the crotch region.

7. The absorbent article according to any one of claims 1 to 5, wherein a plurality of the first elastic portions is provided at intervals in the front-rear direction.

8. The absorbent article according to claim 7, wherein a first elastic portion (61) having a highest contraction force of the first elastic portions is arranged so as not to overlap the absorbent core (50) in the thickness direction.

9. The absorbent article according to any one of claims 1 to 8, wherein
the absorbent body (40) has a plurality of sheet materials extending outside in the front-rear direction relative to the absorbent core (50),
the outer end part (45) of the absorbent body is provided with a sheet non-bonding region (NS) in which at least two sheet materials are not bonded together, and
the sheet non-bonding region (NS), the body non-bonding region (NB), and the first elastic portion (61) are arranged to overlap in the thickness direction at the outer end part (45).

10. The absorbent article according to any one of claims 1 to 9, wherein
the exterior body (15) has a plurality of exterior sheets,
an outside in the front-rear direction relative to an outer end edge of the absorbent body (40) is provided with an exterior non-bonding region (NE) in which at least two exterior sheets of the exterior sheets are not bonded together, and
the exterior non-bonding region (NE), the body non-bonding region (NB), and the first elastic portion (61) are arranged to overlap in the thickness direction at the outer end part (45).

## Patentansprüche

1. Absorbierender Artikel (10), der Folgendes umfasst:
eine Vorn-Hinten-Richtung (L);
eine Breitenrichtung (W) rechtwinklig zu der Vorn-Hinten-Richtung;
einen vorderen Taillenbereich (S1);
einen hinteren Taillenbereich (S2);
einen Schrittbereich (S3), der zwischen dem vorderen Taillenbereich und dem hinteren Taillenbereich angeordnet ist,
einen Saugkörper (40), der einen Saugkern (50) aufweist und angeordnet ist, um den Schrittbereich, den vorderen Taillenbereich und den hinteren Taillenbereich abzudecken;
einen Außenkörper (15), der mindestens in dem vorderen Taillenbereich (S1) und dem hinteren Taillenbereich (S2) auf einer Nicht-Hautoberflächenseite des Saugkörpers (40) angeordnet ist, und
ein elastisches Element (65), dehnbar in der Breitenrichtung, das in dem Außenkörper (15) angeordnet ist, wobei
ein äußerer Endteil (45) einen vorderen Endteil und einen hinteren Endteil einschließt, der vordere Endteil ein Bereich zwischen einem vorderen Endrand (50F) des Saugkerns (50) und einem vorderen Endrand (40F) des Saugkörpers (40) in der Vorn-Hinten-Richtung (L) ist und ein Bereich von einem äußeren Rand (40E) des Saugkörpers (40) zu dem anderen äußeren Rand (40E) in der Breitenrichtung (W) ist,
der hintere Endteil ein Bereich zwischen einem hinteren Endrand (50R) des Saugkerns (50) und einem hinteren Endrand (40R) des Saugkörpers (40) in der Vorn-Hinten-Richtung (L) ist und ein Bereich von einem äußeren Rand (40E) des Saugkörpers (40) zu dem anderen äußeren Rand (40E) in der Breitenrichtung (W) ist,
einer oder beide des vorderen Endteils und des hinteren Endteils des äußeren Endteils (45) in der Vorn-Hinten-Richtung des Saugkörpers (40) mit einem Körper-nichtbindenden Bereich (NB) bereitgestellt ist, in dem der Saugkörper (40) und der Außenkörper (15) nicht verbunden sind,
der äußere Endrand des Körper-nichtbindenden Bereichs (NB) mit dem äußeren Endrand des Saugkörpers (40) in der Vorn-Hinten-Richtung (L) zusammenfällt,
das elastische Element (65) einen ersten elastischen Abschnitt (61) aufweist, der zur Überlappung mit dem Körper-nichtbindenden Bereich (NB) in einer Dickenrichtung angeordnet ist,
an dem äußeren Endteil (45) eine Kontraktionskraft in der Breitenrichtung des Körper-nichtbindenden Bereichs (NB) höher ist als eine Kontraktionskraft in der Breitenrichtung eines benachbarten Bereichs, der sich außen in der Vorn-Hinten-Richtung von einem äußeren Endrand des Körper-nichtbindenden Bereichs (NB) erstreckt,
der absorbierende Artikel (10) mit einem Körper-bindenden Bereich (AB) bereitgestellt ist, in dem der Saugkörper (40) und der Außenkörper (15) verbunden sind,
der Körper-bindende Bereich (AB) außen in der Breitenrichtung (W) in Bezug auf den Körper-nichtbindenden Bereich (NB) an dem äußeren Endteil (45) angeordnet ist und das elastische Element (65) einen dritten elastischen Abschnitt (63) aufweist, der zur Überlappung mit dem Körper-bindenden Bereich (AB) in der Dickenrichtung angeordnet ist, und
der dritte elastische Abschnitt (63) zu der Seite des Schrittbereichs (S3) gebogen ist.

2. Absorbierender Artikel nach Anspruch 1, wobei mindestens ein Teil des Körper-nichtbindenden Bereichs (NB) zur Überlappung mit dem Saugkern (50) in der Vorn-Hinten-Richtung angeordnet ist.

3. Absorbierender Artikel nach Anspruch 2, wobei
der Saugkörper (40) einen mittleren Bereich (CR) aufweist, der ein Bereich ist, der durch Teilen einer Gesamtlänge des Saugkerns (50) in der Breitenrichtung in drei gleiche Teile erhalten wird, und sich an einer Mitte des Saugkerns in der Breitenrichtung befindet, und
der Körper-nichtbindende Bereich (NB) zur Überlappung mit dem mittleren Bereich (CR) in der Vorn-Hinten-Richtung angeordnet ist.

4. Absorbierender Artikel nach Anspruch 2 oder 3, wobei der Körper-nichtbindende Bereich (NB) zur Überlappung in der Vorn-Hinten-Richtung mit einem gesamten Bereich des Saugkerns (50) in der Breitenrichtung angeordnet ist.

5. Absorbierender Artikel nach Anspruch 1, wobei ein Endrand in der Schrittbereichsseite des Körper-nichtbindenden Bereichs (NB) derart angeordnet ist, um nicht mit dem Saugkern (50) in der ebenen Ansicht einer Dickenrichtung (T) zu überlappen.

6. Absorbierender Artikel nach Anspruch 5, wobei
das elastische Element (65) einen zweiten elastischen Abschnitt (62) aufweist, der auf der Seite des Schrittbereichs (S3) in Bezug auf den Körper-nichtbindenden Bereich (NB) und außerhalb in der Vorn-Hinten-Richtung in Bezug auf den Saugkern (50) angeordnet ist, und
der zweite elastische Abschnitt (62) zu der Seite des Schrittbereichs gebogen ist.

7. Absorbierender Artikel nach einem der Ansprüche 1 bis 5, wobei eine Vielzahl der ersten elastischen Abschnitte in Abständen in der Vorn-Hinten-Richtung bereitgestellt ist.

8. Absorbierender Artikel nach Anspruch 7, wobei ein erster elastischer Abschnitt (61), der eine höchste Kontraktionskraft der ersten elastischen Abschnitte aufweist, derart angeordnet ist, um nicht mit dem Saugkern (50) in der Dickenrichtung zu überlappen.

9. Absorbierender Artikel nach einem der Ansprüche 1 bis 8, wobei
der Saugkörper (40) eine Vielzahl von Lagenmaterialien aufweist, die sich außen in der Vorn-Hinten-Richtung in Bezug auf den Saugkern (50) erstrecken,
der äußere Endteil (45) des Saugkörpers mit einem Lagen-nichtbindenden Bereich (NS) bereitgestellt ist, in dem mindestens zwei Lagenmaterialien nicht miteinander verbunden sind, und
der Lagen-nichtbindende Bereich (NS), der Körper-nichtbindende Bereich (NB) und der erste elastische Abschnitt (61) zur Überlappung in der Dickenrichtung an dem äußeren Endteil (45) angeordnet sind.

10. Absorbierender Artikel nach einem der Ansprüche 1 bis 9, wobei
der Außenkörper (15) eine Vielzahl von Außenlagen aufweist,
eine Außenseite in der Vorn-Hinten-Richtung in Bezug auf einen äußeren Endrand des Saugkörpers (40) mit einem äußeren nichtbindenden Bereich (NE) bereitgestellt ist, in dem mindestens zwei Außenlagen der Außenlagen nicht miteinander verbunden sind, und
der äußere nichtbindende Bereich (NE), der Körper-nichtbindende Bereich (NB) und der erste elastische Abschnitt (61) zur Überlappung in der Dickenrichtung an dem äußeren Endteil (45) angeordnet sind.

## Revendications

1. Article absorbant (10), comportant :
une direction allant dans le sens avant-arrière (L) ;
une direction allant dans le sens de la largeur (W) orthogonale par rapport à la direction allant dans le sens avant-arrière ;
une région avant au niveau de la taille (S1) ;
une région arrière au niveau de la taille (S2) ;
une région au niveau de l'entrejambe (S3) agencée entre la région avant au niveau de la taille et la région arrière au niveau de la taille ;
un corps absorbant (40) ayant une partie centrale absorbante (50) et agencé pour enjamber la région au niveau de l'entrejambe, la région avant au niveau de la taille et la région arrière au niveau de la taille ;
un corps extérieur (15) agencé dans au moins la région avant au niveau de la taille (S1) et la région arrière au niveau de la taille (S2) sur un côté de surface non orientée vers la peau du corps absorbant (40) ; et
un élément élastique (65) en mesure de s'étirer dans la direction allant dans le sens de la largeur agencé dans le corps extérieur (15), dans lequel
une partie d'extrémité extérieure (45) comprend une partie d'extrémité avant et une partie d'extrémité arrière,
la partie d'extrémité avant est une région entre un bord d'extrémité avant (50F) de la partie centrale absorbante (50) et un bord d'extrémité avant (40F) du corps absorbant (40) dans la direction allant dans le sens avant-arrière (L) et est une région allant d'un bord extérieur (40E) du corps absorbant (40) à l'autre bord extérieur (40E) dans la direction allant dans le sens de la largeur (W),
la partie d'extrémité arrière est une région entre un bord d'extrémité arrière (50R) de la partie centrale absorbante (50) et un bord d'extrémité arrière (40R) du corps absorbant (40) dans la direction allant dans le sens avant-arrière (L) et est une région allant d'un bord extérieur (40E) du corps absorbant (40) à l'autre bord extérieur (40E) dans la direction allant dans le sens de la largeur (W),
une ou les deux parmi la partie d'extrémité avant et la partie d'extrémité arrière de la partie d'extrémité extérieure (45) dans la direction allant dans le sens avant-arrière du corps absorbant (40) sont dotées d'une région de non-liage de corps (NB) dans laquelle le corps absorbant (40) et le corps extérieur (15) ne sont pas liés, le bord d'extrémité extérieur de la région de non-liage de corps (NB) coïncide avec le bord d'extrémité extérieur du corps absorbant (40) dans la direction allant dans le sens avant-arrière (L),
l'élément élastique (65) a une première partie élastique (61) agencée pour chevaucher la région de non-liage de corps (NB) dans une direction allant dans le sens de l'épaisseur,
au niveau de la partie d'extrémité extérieure (45), la force de contraction dans la direction allant dans le sens de la largeur de la région de non-liage de corps (NB) est supérieure à la force de contraction dans la direction allant dans le sens de la largeur d'une région adjacente s'étendant à l'extérieur dans la direction allant dans le sens avant-arrière depuis un bord d'extrémité extérieur de la région de non-liage de corps (NB),
l'article absorbant (10) est doté d'une région de liage de corps (AB) dans laquelle le corps absorbant (40) et le corps extérieur (15) sont liés,
la région de liage de corps (AB) est agencée à l'extérieur dans la direction allant dans le sens de la largeur (W) par rapport à la région de non-liage de corps (NB) au niveau de la partie d'extrémité extérieure (45), et
l'élément élastique (65) a une troisième partie élastique (63) agencée pour chevaucher la région de liage de corps (AB) dans la direction allant dans le sens de l'épaisseur, et
la troisième partie élastique (63) est courbe par rapport au côté de la région au niveau de l'entrejambe (S3).

2. Article absorbant selon la revendication 1, dans lequel au moins une partie de la région de non-liage de corps (NB) est agencée pour chevaucher la partie centrale absorbante (50) dans la direction allant dans le sens avant-arrière.

3. Article absorbant selon la revendication 2, dans lequel
le corps absorbant (40) a une région centrale (CR) qui est une région obtenue en divisant une longueur totale de la partie centrale absorbante (50) dans la direction allant dans le sens de la largeur en trois parties égales et qui est située au niveau d'un centre de la partie centrale absorbante dans la direction allant dans le sens de la largeur, et
la région de non-liage de corps (NB) est agencée pour chevaucher la région centrale (CR) dans la direction allant dans le sens avant-arrière.

4. Article absorbant selon la revendication 2 ou la revendication 3, dans lequel la région de non-liage de corps (NB) est agencée pour chevaucher, dans la direction allant dans le sens avant-arrière, toute une région de la partie centrale absorbante (50) dans la direction allant dans le sens de la largeur.

5. Article absorbant selon la revendication 1, dans lequel un bord d'extrémité dans le côté de la région au niveau de l'entrejambe de la région de non-liage de corps (NB) est agencé de manière à ne pas chevaucher la partie centrale absorbante (50) dans une vue en plan d'une direction allant dans le sens de l'épaisseur (T).

6. Article absorbant selon la revendication 5, dans lequel
l'élément élastique (65) a une deuxième partie élastique (62) agencée sur le côté de la région au niveau de l'entrejambe (S3) par rapport à la région de non-liage de corps (NB) et à l'extérieur dans la direction allant dans le sens avant-arrière (50), et
la deuxième partie élastique (62) est courbe par rapport au côté de la région au niveau de l'entrejambe.

7. Article absorbant selon l'une quelconque des revendications 1 à 5, dans lequel une pluralité des premières parties élastiques est mise en œuvre selon des intervalles dans la direction allant dans le sens avant-arrière.

8. Article absorbant selon la revendication 7, dans lequel une première partie élastique (61) ayant une force de contraction la plus élevée des premières parties élastiques est agencée de manière à ne pas chevaucher la partie centrale absorbante (50) dans la direction allant dans le sens de l'épaisseur.

9. Article absorbant selon l'une quelconque des revendications 1 à 8, dans lequel
le corps absorbant (40) a une pluralité de matériaux en feuilles s'étendant vers l'extérieur dans la direction allant dans le sens avant-arrière par rapport à la partie centrale absorbante (50),
la partie d'extrémité extérieure (45) du corps absorbant est dotée d'une région de non-liage de feuilles (NS) dans laquelle au moins deux matériaux en feuilles ne sont pas liés ensemble, et
la région de non-liage de feuilles (NS), la région de non-liage de corps (NB) et la première partie élastique (61) sont agencées pour se chevaucher dans la direction allant dans le sens de l'épaisseur au niveau de la partie d'extrémité extérieure (45).

10. Article absorbant selon l'une quelconque des revendications 1 à 9, dans lequel
le corps extérieur (15) a une pluralité de feuilles extérieures, une partie extérieure dans la direction allant dans le sens avant-arrière par rapport à un bord d'extrémité extérieur du corps absorbant (40) est dotée d'une région de non-liage extérieure (NE) dans laquelle au moins deux feuilles extérieures des feuilles extérieures ne sont pas liées ensemble, et
la région de non-liage extérieure (NE), la région de non-liage de corps (NB) et la première partie élastique (61) sont agencées pour se chevaucher dans la direction allant dans le sens de l'épaisseur au niveau de la partie d'extrémité extérieure (45).
